(19) Europäisches Patentamt
European Patent Office
Office européen des brevets

(11) Publication number: **0 530 016 B1**

(12) # EUROPEAN PATENT SPECIFICATION

(45) Date of publication of patent specification: **02.08.95**

(51) Int. Cl.$^6$: **C07D 211/70**, C07D 409/04, A61K 31/445

(21) Application number: **92307790.3**

(22) Date of filing: **26.08.92**

(54) **Piperidine derivative and pharmaceutical composition containing it.**

(30) Priority: **27.08.91 JP 214967/91**

(43) Date of publication of application:
**03.03.93 Bulletin 93/09**

(45) Publication of the grant of the patent:
**02.08.95 Bulletin 95/31**

(84) Designated Contracting States:
**CH DE FR GB IT LI**

(56) References cited:
**EP-A- 0 294 183**
**EP-A- 0 370 712**
**EP-A- 0 371 805**
**EP-A- 0 479 601**
**US-A- 4 356 184**

(73) Proprietor: **Ajinomoto Co., Inc.**
**No. 15-1, Kyobashi 1-chome,**
**Chuo-ku**
**Tokyo 104 (JP)**

(72) Inventor: **Minomura, Masafumi, c/o Central Research Lab.**
**Ajinomoto Co., Inc.,**
**1-1 Suzuki-cho,**
**Kawasaki-ku**
**Kawasaki-shi,**
**Kanagawa-ken (JP)**
Inventor: **Kanematu, Akira, c/o Central Research Lab.**
**Ajinomoto Co., Inc.,**
**1-1 Suzuki-cho,**
**Kawasaki-ku**
**Kawasaki-shi,**
**Kanagawa-ken (JP)**
Inventor: **Kaida, Masato, c/o Central Research Lab.**
**Ajinomoto Co., Inc.,**
**1-1 Suzuki-cho,**
**Kawasaki-ku**
**Kawasaki-shi,**
**Kanagawa-ken (JP)**
Inventor: **Dohmoto, Hideki, c/o Central Research Lab.**
**Ajinomoto Co., Inc.,**
**1-1 Suzuki-cho,**
**Kawasaki-ku**
**Kawasaki-shi,**
**Kanagawa-ken (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 530 016 B1

Inventor: **Yoshimoto, Ryota, c/o Central Research Lab.**
**Ajinomoto Co., Inc.,**
**1-1 Suzuki-cho,**
**Kawasaki-ku**
**Kawasaki-shi,**
**Kanagawa-ken (JP)**
Inventor: **Shoji, Masataka, c/o Central Research Lab.**
**Ajinomoto Co., Inc.,**
**1-1 Suzuki-cho,**
**Kawasaki-ku**
**Kawasaki-shi,**
**Kanagawa-ken (JP)**

(74) Representative: **Nicholls, Kathryn Margaret et al**
**MEWBURN ELLIS**
**York House**
**23 Kingsway**
**London WC2B 6HP (GB)**

## Description

The present invention relates to a piperidine derivative or pharmaceutically acceptable salt thereof, to a pharmaceutical preparation containing the derivative or salt and to methods of preparing and using the derivative or salt.

Hypertension is a common disorder in the modern world. In Japan alone it is estimated that around 13 million people suffer from it. Hypertension is particularly prevalent in the old and its incidence increases with age. Increasing attention has been paid to hypertension in the elderly as it increases the risk of severe heart and cerebral disorders such as cardiac infarction and apoplexy.

In recent years calcium antagonists, angiotensin converting enzyme inhibitors and α-1 blockers have been the drugs of first choice for treatment of hypertension. However, the benefits of these drugs are questionable when their side effects and safety are taken into consideration.

According to one aspect of the invention there is provided a piperidine derivative of formula (I):

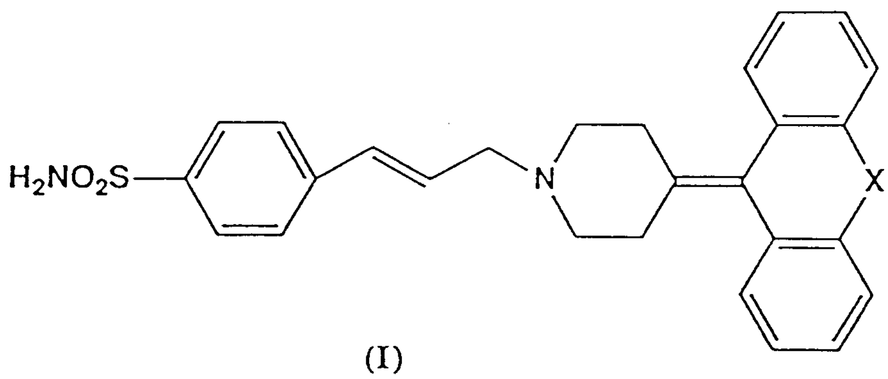

(I)

wherein X is $-CH=CH-$, $-CH_2CH_2-$ or $-CH_2S-$ ;
or a pharmaceutically acceptable salt thereof. Such compounds have been found to have good antihypertensive activity and may be readily produced on an industrial scale.

According to a second aspect of the invention there is provided a pharmaceutical composition comprising the piperidine derivative or salt of the first aspect and a pharmaceutically acceptable excipient, diluent or carrier.

According to a third aspect of the invention there is provided the use of a derivative or salt of the first aspect in the manufacture of a medicament for treatment or prevention of hypertension in a mammalian, preferably human, patient.

Additionally, according to a still further aspect there is provided a method for the production of a derivative of formula (I) or a pharmaceutically acceptable salt thereof comprising at least one of the steps:

(A) reacting a compound of formula (II)

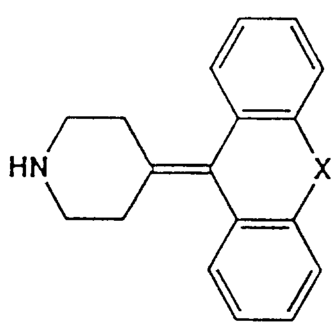

(II)

wherein X is as defined above, and a compound of formula (1)

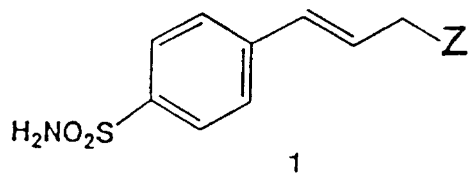

1

wherein Z is a leaving group; and

(B) reacting a derivative of formula (I) with an appropriate acid, thereby to form a pharmaceutically acceptable salt of the derivative.

The skilled man will understand that the leaving group Z may be any which may be displaced by nucleophilic substition. Examples include atoms such as a halogen atom, in particular iodine, or groups of atoms such as a tosyl group.

According to a fifth aspect of the invention there are provided intermediates in the production of a derivative of formula (I), the intermediates being a compound of formula (1) as illustrated above, or 4-aminosulfonylcinnamyl alcohol of formula (2) as illustrated below which is a precursor of the compound of formula (1).

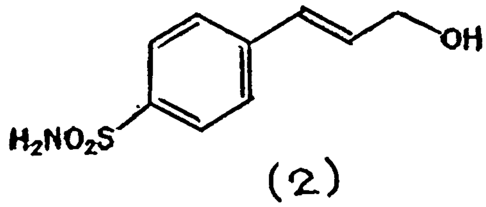

(2)

Suitable synthetic routes for the production of a derivative of formula (I) are illustrated below in Schemes 1 and 2.

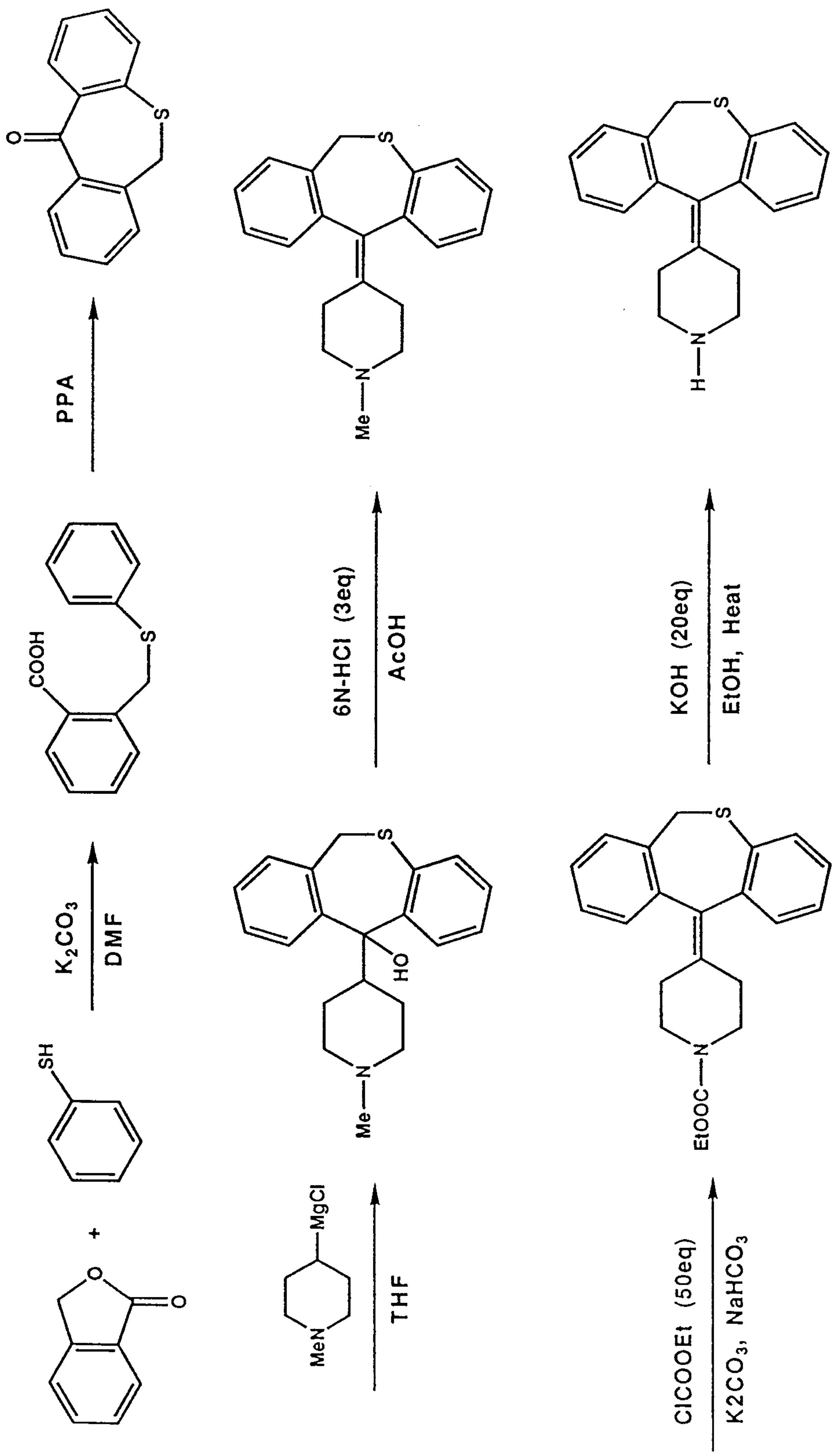
PPA
$K_2CO_3$
DMF
6N-HCl (3eq)
AcOH
THF
KOH (20eq)
EtOH, Heat
ClCOOEt (50eq)
K2CO3, NaHCO3

SCHEME 1

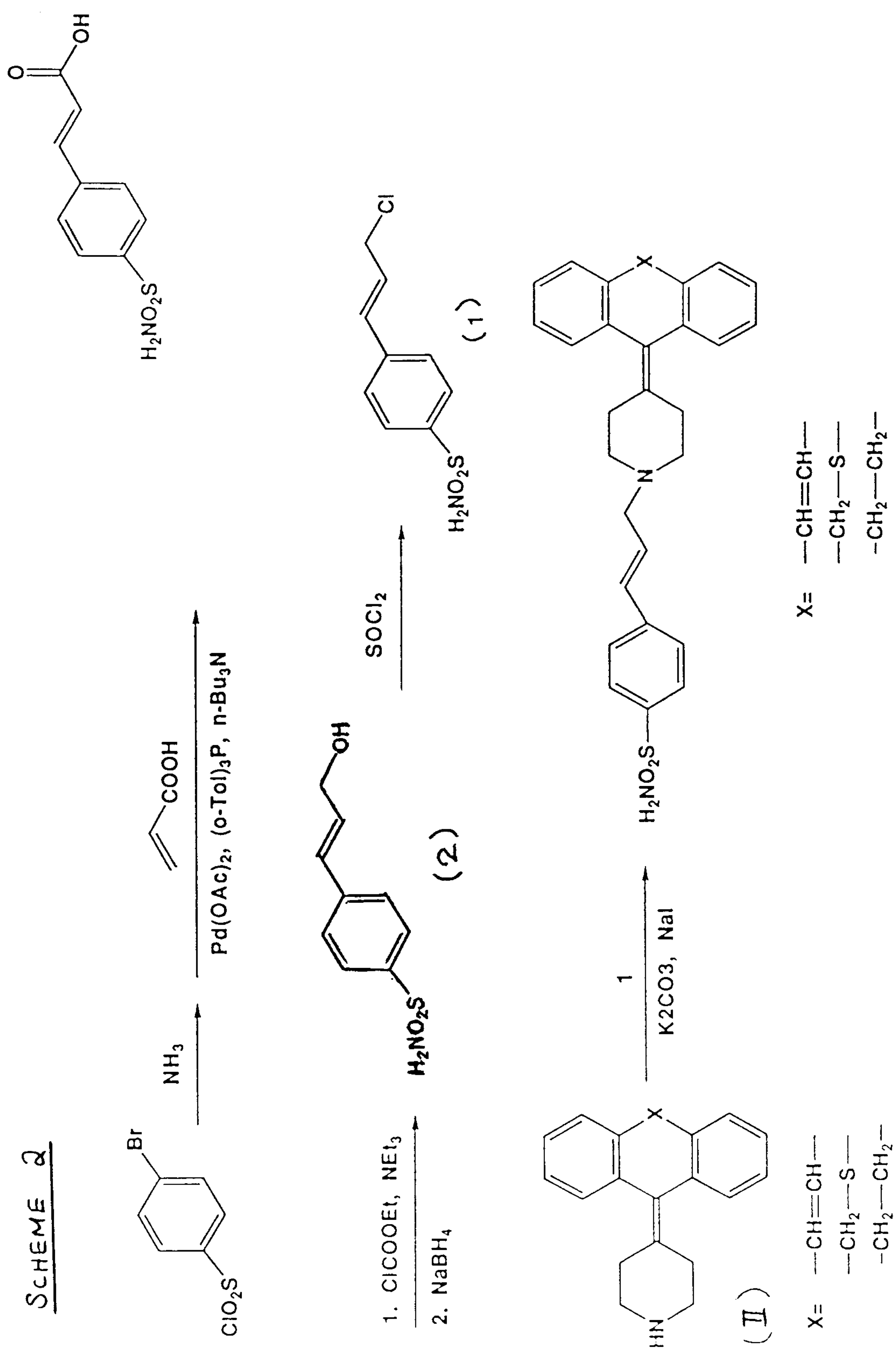


Scheme 1 indicates how an intermediate of formula (II) wherein X is $-CH_2S-$ may be prepared. This compound may also be prepared by the method of Hulinska et al (Collect.Czech. Chem. Commun. 54, 1388 (1989). The compound of formula (II) wherein X is -CH=CH- may be prepared by the method of Engelhardt et al (J. Med.Chem., 8,829 (1965)). When X is $-CH_2CH_2-$ compound (II) may be prepared by the method of Japanese patent laid-open no. 3-128354.

Scheme 2 indicates how a compound of formula (2) may be prepared and converted into a compound of formula (1) which is subsequently reacted with a compound of formula (II) wherein X is any one of $-CH_2CH_2-$, $-CH=CH-$ or $-CH_2S-$to produce a piperidine derivative of formula (I).

Derivatives of formula (I) produced by the method illustrated in Scheme 2 or by any other route may be treated with a suitable acid, such as hydrochloric acid to form a pharmaceutically acceptable acid addition salt. Suitable acids for such a purpose will be apparent to those of skill in the art.

The piperidine derivative or salt of the present invention when for use as an antihypertensive may be administered by an oral or parenteral route. The appropriate dose will depend on the age, body weight and condition of the patient as well as on the route by which the derivative or salt is administered. In general where the derivative or salt is administered orally a daily dose in the range of about 0.01 to about 2000 mg/kg is appropriate; if the derivative is administered parenterally then a does of about 0.01 to 1000 mg/kg may be suitable.

The piperidine derivative or salt of the present invention may be formulated in conventional preparation forms, for example as tablet, powders, capsules, solutions, sugar-coated tablets or depots, which may be prepared in a conventional manner using conventional methods. For example, tablets can be obtained by mixing the piperidine derivative or salt of the present invention with known auxiliary substances, for example, inactive diluents (eg. lactose, calcium carbonate or calcium phosphate), binders (eg. arabic gum, corn starch or gelatine), swelling agents (eg. arginic acid, corn starch, pregelatinated starch), sweeteners (eg. sucrose or saccharine), flavours (eg. peppermint, Gaultheria adenothrix oil or cherry), lubricating and wetting agents (eg. magnesium stearate, talc or carboxymethyl cellulose).

Embodiments of the present invention are described below by way of example only. In the examples: silica gel thin layer chromatography was used for the chromatographic analysis of samples unless stated otherwise; mass spectroscopy (MS) was performed in the field desorption (FD) mode (M/Z) and nuclear magnetic resonance (NMR) spectra were measured using tetramethylsilane as an internal standard and deutero-chloroform as the solvent.

## EXAMPLES

### Example 1

**1-(4-Aminosulfonylcinnamyl)-4-(5H-dibenzo[a,d]cyclohepten-5-ylidene)piperidine:**

**a)4-aminosulfonylcinnamic acid:**

A solution of 2.36g (10.0 mmol) of 4-bromobenzene sulfonamide, 1.44g (20.0 mmol) of acrylic acid, 3.71g (20.0 mmol) of tributylamine, 44.9mg (0.20 mmol) of palladium acetate and 365mg (1.20 mmol) of tris(2-methyphenyl)phosphine in 100mL of acetonitrile was stirred in an argon atmosphere and refluxed at 100 °C overnight in an oil bath. After reaction, the reaction mixture was filtered to remove any solids. Then the solvent was removed under reduced pressure, and the residue was crystallised with 1N-HCl (100mL). The resulting white crystals were obtained by filtration.

Amount yielded: 1.89g (8.32 mmol)

% Yield: 83%

TLC($CHC1_3$:MeOH = 9:1):Rf = 0.05

**b)4-Aminosulfonylcinnamyl alcohol:**

1.00g (4.41 mmol) of 4-aminosulfonylcinnamic acid and 0.485g (4.80 mmol) of triethylamine were dissolved in 50mL of tetrahydrofuran (THF) and 50mL of dimethyl formamide (DMF). Then 0.521g (4.80 mmol) of ethyl chloroformate was added carefully to this solution at-10 °C, and stirred for 40min. After the reaction, the reaction mixture was filtered to remove triethylamine HCl salt at -10 °C After filtration, a solution of 0.493g (13.0 mmol) of sodium borohydride in THF(50mL) was added portionwise to the filtrate at -10 °C. After stirring for 1hr, the solution was neutralised with 1N-HCl and the solvent was removed under reduced pressure. 50mL of water and 50mL of $CHC1_3$ were added to the residue and then the organic layer was separated. After drying with anhydrous $MgSO_4$ the solvent was removed under reduced pressure to yield 4-aminosulfonylcinnamyl alcohol.

Amount yielded: 0.273g (1.28mmol)

% Yield: 29%

TLC($CHC1_3$:MeOH = 9:1)Rf = 0.20

MS: 213(M + )
NMR:
4.29(2H,d,J = 5.3Hz),6.43-6.55(1H,m), 6.67(1H,d,J = 16.0Hz), 7.52 (2H,d,J = 8.4Hz), 7.84(2H,d,J = 8.7Hz).

**c)4-Aminosulfonylcinnamyl chloride:**

0.728g (3.42mmol) of 4-aminosulfonylcinnamyl alcohol was added to a solution of 5ml of thionyl chloride and 30ml of $CHCl_3$ at 0 °C. After stirring for 4 hours, the thionyl chloride and chloroform were removed under reduced pressure, and then 50ml of $CHCl_3$ was added to the residue. The organic layer was washed with water and dried with anhydrous $MgSO_4$. The solvent was removed under reduced pressure to yield 4-aminosulfonylcinnamyl chloride.

Amount yielded: 670mg (2.89mmol)
% Yield: 85%
TLC(CHC$1_3$:MeOH = 9:1)Rf = 0.34
MS:231(M + )
NMR:
4.26(2H,d,J = 6.9Hz) 6.35-6.55(1H,m) 6.71 (1H,d,J = 15.9Hz) 7.52(2H,d,J = 8.4Hz) 7.90(2H,d,J = 8.7Hz)

**d)1-(4-Aminosulfonylcinnamyl)-4-(5H-dibenzo[a,d] cyclohepten-5-ylidene)piperidine:**

Then a mixture of 0.500g (2.16mmol) 4-aminosulfonylcinnamyl chloride, 0.590g (2.16mmol) of 4-(5H-dibenzo[a,d]cyclohepten-5-ylidene)piperidine and 0.596g(4.32mmol) of anhydrous $K_2CO_3$ in 75mL of methyl isobutyl ketone (MIBK) was stirred at 70 °C overnight. After the reaction, the reaction mixture was washed with 50mL of water and the organic layer was separated and extracted with EtOAc. After drying with anhydrous $MgSO_4$, the solvent was removed under reduced pressure. The residue was purified by silica gel column chromatography (eluant: MeOH/$CHCl_3$ = 1/50) to yield 1-(4-aminosulfonylcinnamyl)-4-(5H-dibenzo-[a,d]cyclohepten-5-ylidene)piperidine.

Amount yielded: 0.680g (1.45mmol)
% Yield: 67%
TLC($CHCl_3$):MeOH = 9:1):Rf = 0.48
MS:468(M + )
NMR:
2.19(4H,m),2.38(2H,m),2.62(2H,m),3.14(2H,d),6.30-6.52(2H,m),6.92(2H,s),7.18-7.35(8H,m),7.42(2H,d)-7.82(2H,d)

**Example 2**

**11-[1-(4-Aminosulfonylcinnamyl)-4-piperidinylidene]-6,11-dihydrodibenzo[b,e]thiepin:**

**a)2-Phenylthiomethylphenyl-1-carboxylic acid:**

25.0g(0.227 mmol) of thiophenol, 30.5g (0.227 mmol) of phthalide and 68.9g (0.499 mol) of anhydrous $K_2CO_3$ were dissolved in 200mL of DMF, and stirred for 4 hours at 100 °C. According to normal procedure, the organic layer was separated and extracted with $CH_2Cl_2$. Then the organic layer was washed with water, 1N-HC1, saturated $NaHCO_3$ and brine. After drying with anhydrous $MgSO_4$, the solvent was removed under reduced pressure to yield 2-phenylthiomethylphenyl-1-carboxylic acid.

Amount yielded: 41.2g (0.169 mol)
% Yield: 74%
TLC ($CHCl_3$:MeOH = 9:1):Rf = 0.40

b)6,11-dihydrodibenzo[b,e]thiepin-11-one:

400g of polyphosphoric acid was added to 41.2g (0.169 mmol) of 2-phenylthiomethylphenyl-1-carboxylic acid and stirred with a mechanical stirrer for 3 hours at 120 °C. According to conventional procedure, the organic layer was separated and extracted with $CH_2Cl_2$. Then the organic layer was washed with water, 1N-HC1, saturated $NaHCO_3$ and brine. After drying with anhydrous $MgSO_4$, the solvent was removed under reduced pressure. The residue was purified with silica gel chromatography (eluant:n-Hexane/EtOAc = 3:1) to yield 6,11-dihydrodibenzo[b,e]thiepin-11-one.

Amount yielded:37.0g (0.164 mol)
% Yield: 97%
TLC($CHCl_3$:MeOH = 9:1):Rf = 0.81

**c)11-(1-methyl-4-piperidinyl)-11Hydroxy-6,11-dihydrodibenzo[b,e]thiepin:**

The Grignard reagent was prepared in the usual manner using 6.9g of magnesium and 25.7g (0.193 mol) of 1-methyl-4-piperidine in 170mL of anhydrous THF using $I_2$ as initiator. A solution of 9.0g (39.8 mmol) of 6,11-dihydrodibenzo[b,e]thiepin-11-one and 160mL of THF was added dropwise at room temperature to the mixture and was stirred at reflux for 2 hours. The organic material was extracted with $CHCl_3$ by an ordinary procedure. Then the organic layer was washed with water, 1N-HCl, saturated $NaHCO_3$ and brine. After drying with anhydrous $MgSO_4$, the solvent was removed under reduced pressure. The residue was purified with silica gel column chromatography (eluant: $CHCl_3$/MeOH = 30:1) to yield 11-(1-methyl-4-piperidinyl)-11-Hydroxy-6,11-dihydrodibenzo[b,e]thiepin.
Amount yielded: 47.1g (0.145mol)
% Yield: 89%
MS: 324(M + )
TLC ($CHCl_3$:MeOH = 9:1)Rf = 0.20
NMR
1.2-1.5(2H,m),2.6-2.8(2H,m),2.0-2.2(2H,m),2.33 (3H,s),2.95(2H,t),3.52(1H,m),3.83(1H,d), 4.62(1H,d),7.0-7.3(7H,m),7.8-7.9(1H,m)

**d)11-(1-methyl-4-piperidinylidene)-6,11-dihydrodibenzo[b,e]thiepin:**

50mL of 4N-HC1 was added to a solution of 45.0g (0.139mol) of 11-(1-methyl-4-piperidinyl)-11-Hydroxy-6,11-dihydrodibenzo[b,e]thiepin in 250mL of acetic acid and the mixture was heated at 100 ° C overnight. According to ordinary procedure, the organic material was extracted with $CH_2C1_2$. Then the organic layer was washed with water, 1N-HC1, saturated $NaHCO_3$ and brine. After drying with anhydrous $MgSO_4$, the solvent was removed under reduced pressure. The residue was purified with silica gel column chromatography (eluant:$CHC1_3$/MeOH = 50:1) to yield 11-(1-methyl-4-piperidinylidene)-6,11-dihydrodibenzo[b,e]thiepin.
Amount yielded:37.0g(0.121 mol)
% Yield: 87%
MS: 324(M + )
TLC ($CHCl_3$:MeOH = 9:1):Rf = 0.41
NMR:
2.1-2.2(2H,m)2.2-2.4(2H,m),2.29(3H,s),2.5-2.6(2H,m), 2.6-2.7(2H,m),3.40(1H,d),4.95(1H,s),7.0-7.1(4H,m)-,7.2-7.4(7H,m)

**e)11-(4-piperidinylidene-6,11-dihydrodibenzo[b,e]thiepin:**

300g (2.76 mol) of ethyl chloroformate was added portionwise to a mixture of 39.0g (0.127 mol) of 11-(1-methyl-4-piperidinylidene)-6,11-dihydrodibenzo[b,e]thiepin, 17.5g (0.127 mol) of anhydrous $K_2CO_3$, and 10.6g (0.127 mol) of $NaHCO_3$ at room temperature, and the mixture was stirred at 70 ° C for 3 hr on an oil bath. Then the reaction mixture was cooled to room temperature and an additional 8.7g (0.063 mol) of anhydrous $K_2CO_3$ and 5.3g (0.063 mol) of $NaHCO_3$ were added to the mixture, and the mixture was again heated at 70 ° C for 3 hr. The mixture was cooled to room temperature, filtered to remove the salts, and unreacted ethyl chloroformate was removed under reduced pressure. Then the organic material was extracted with $CH_2Cl_2$, washed with water, saturated $NaHCO_3$ and brine. After drying with anhydrous $MgSO_4$, the solvent was removed under reduced pressure to yield 11-(1-ethoxy-4-piperidinylidene)-6,11-dihydrodibenzo[b,e]thiepin as a crude product. This was dissolved in 400 mL of ethanol and 140g (2.54 mol) of powdered KOH was added to this at room temperature. After a vigorous reaction was complete, the solution was stirred at reflux overnight. The flask was cooled to room temperature, and the solvent was removed under reduced pressure. According to ordinary procedure, the organic material was extracted with $CH_2Cl_2$, washed with water, saturated $NaHCO_3$ and brine. After drying with anhydrous $MgSO_4$, the solvent was removed under reduced pressure to yield 11-(4-piperidinylidene)-6,11-dihydrodibenzo[b,e]thiepin.
Amount yielded: 28.0g(0.095 mol)
%Yield: 75%
MS: 293 (M + )

TLC($CHCl_3$:MeOH = 4:1): Rf = 0.32
NMR:
2.10(2H,dt), 2.42(2H,t), 2.73(1H,m), 2.8-3.1(4H,m), 3.40(1H,d), 4.97(1H,d), 7.0-7.1(4H,m), 7.2-7.4(7H,m)

**f)11-[1-(4-Aminosulfonylcinnamyl)-4-piperidinylidene]-6,11-dihydrodibenzo[b,e]thiepin:**

A mixture of 1.00g (4.3 mmol) of 4-aminosulfonylcinnamyl chloride, 1.51g (5.18 mmol) of 11-(1-methyl-4-piperidinylidene)-6,11-dihydrodibenzo[b,e]thiepin, 0.89g (6.5 mmol) of anhydrous $K_2CO_3$ and 0.89g (6.5 mmol) of sodium iodide was stirred at 110 °C over night. According to ordinary procedure, the organic material was extracted with $CHCl_3$. Then the organic layer was washed with water, 1N-HCl, saturated $NaHCO_3$ and brine. After drying with anhydrous $MgSO_4$, the solvent was removed under reduced pressure. The residue was purified with silica gel column chromatography (eluant: $CHCl_3$/MeOH = 50:1) to yield 11-[1-(4-Aminosulfonylcinnamyl)-4-piperidinylidene]-6,11-dihydrodibenzo[b,e]thiepin.

Amount yielded: 1.83g(3.76 mmol)
Yield: 88%
MS: 488 (M + )
TLC($CHCl_3$:MeOH = 9:1):Rf = 0.40
NMR:
2.19(4H,m), 2.38(2H,m), 2.62(2H,m), 3.14(2H,m), 6.30-6.52(2H,m), 6.92(2H,s), 7.18-7.35(8H,m), 7.42-(2H,d), 7.82(2H,d)

Test Example 1

Two male spontaneously hypertensive rats (body weight 400-440g) and in which hypertension was confirmed were used as test animals. These were obtained from Charles River Japan (Kanagawa, Japan) and were maintained in a pathogen free state until used.

An aqueous solution of the derivative prepared in Example 1 above in physiological saline containing 2.5% Nicolle TM and 2.5% ethanol was intravenously administered by bolus injection in a does of 1ml per 1 kg body weight. Systolic blood pressure (SBP) after administration was measured by an indirect (tail-cuff) method. The antihypertensive effect was shown as a reduction in blood pressure (mmHg) from the basal values.

The results are shown below.

Table 1

| Test Compound | dose(iv) (mg/kg) | dSBP (mmHg) | |
|---|---|---|---|
| | | 0.5hr | 4hr |
| (Example 1) | 10 | -83 | -95 |

Test Example 2

Four male spontaneously hypertensive rats (body weight 400-440g) and in which hypertension was confirmed were used. These were obtained from Charles River Japan (Kanagawa, Japan and were maintained in a pathogen free state until used.

A 5% arabic gum aqueous suspension of each sample was administered orally in a dose of 1ml per kg body weight. Systolic blood pressure after administration was measured by an indirect (tail-cuff) method. Antihypertensive effect was shown as a reduction in blood pressure (mmHg) from the basal values.

The results are shown below.

Table 2

| Test Compound | dose(po) (mg/kg) | dSBP (mmHg) | |
|---|---|---|---|
| | | 2hr | 6hr |
| (Example 1)<br>(Example 2) | 6<br>6 | -37<br>-31 | -48<br>-43 |

Test Example 3

Blood was obtained from rabbit and was treated with 3.8% sodium citrate. Platelet rich plasma (PRP) was prepared by centrifugation at 1200 rpm for 10 min at room temperature. The platelet aggregation induced by adenosine diphosphate (ADP) (1.5μM) and by a combination of ADP (1.5μM) and serotonin (5μM) was measured. Then the concentration of piperidine derivative required to produce 50% inhibition of the increased platelet aggregation caused by serotonin compared with the control was calculated as IC50.

The results are shown below.

Table 3

| Test Compound | pIC50 |
|---|---|
| (Example 1)<br>(Example 2) | 6.7<br>6.6 |

Test Example 4

The ability of the sample to block voltage-sensitive calcium channels was determined by the relaxing effects of cumulative concentrations of the sample on the rat aorta pre-contracted by depolarization (50 mmol/l KCl). The concentration of piperidine derivative producing 50% relaxation of the aortic strips contracted with $K^+$ was calculated as the IC50.

The results are shown below.

Table 4

| Test Compound | pIC50 |
|---|---|
| (Example 1) | 8.4 |

Test Example 5

The safety of the derivatives produced in Examples 1 and 2 was examined compared with that of 1-cyclohexyl-4-[4-(5H-dibenzo[a,d]cyclohepten-5-ylidene)piperidinyl]butane hydrochloride and 1-cinnamyl-4-(5H-dibenzo[a,d]cyclohepten-5-ylidene)piperidine hydrochloride, which were previously disclosed in EPO371805 as Example 10 and Example 23 respectively.

As test animals, five to six male Sprague-Dawley (SD) rats (initial body weight 92.9-118.7g) were used.

A 5% arabic gum aqueous suspension of each sample was administered orally once a day for 4 weeks. The number of rats surviving at different times after the start of the experiment was as follows.

Table 5

| Test Compound | dose(po) (mg/kg) | Day of treatment periods 2 | 4 | 8 | 16 | 28 |
|---|---|---|---|---|---|---|
| 1-Cyclohexyl-4-(4-5H-dibenzo[a,d]cyclohepten-5--ylidene)-1-piperidine hydrochloride | 30 | 6/6 | 6/6 | 4/6 | 3/6 | 0/6 |
| 1-Cinnamyl-4-(5H-dibenzo[a,d]cyclohepten-5--ylidene)piperidine hydrochloride | 15 | 5/5 | 4/5 | 3/5 | | |
| ( Example 1) | 60 | 6/6 | 6/6 | 6/6 | 6/6 | 6/6 |
| ( Example 2) | 60 | 6/6 | 6/6 | 6/6 | 6/6 | 6/6 |

## Claims

1. A piperidine derivative of formula (I):

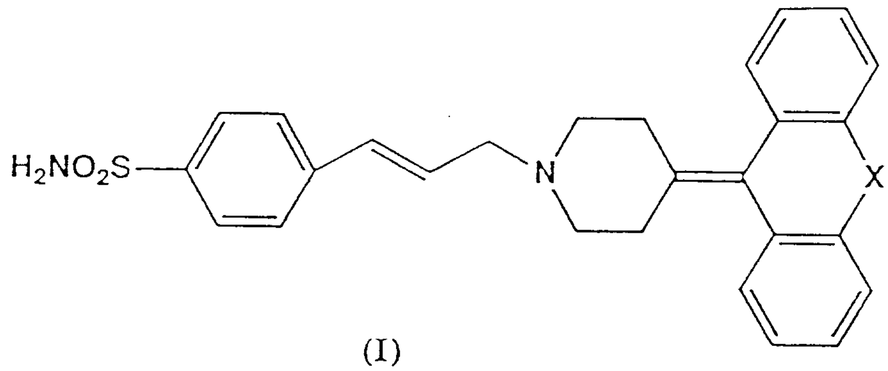

(I)

wherein X is -CH=CH-, $-CH_2CH_2-$ or $-CH_2S-$ ;
or a pharmaceutically acceptable salt thereof.

2. A derivative or salt according to claim 1 for pharmaecutical use.

3. A pharmaceutical composition comprising a derivative or salt according to claim 1 and a pharmaceutically acceptable excipient, diluent or carrier.

4. Use of a derivative or salt according to claim 1 in the manufacture of a medicament for prevention or treatment of hypertension.

5. A use according to claim 4 wherein the medicament is for oral administration.

6. A use according to claim 4 wherein the medicament is for administration by a parenteral route.

7. A use according to claim 5 wherein the medicament is a dosage form suitable for administration of the derivative or salt in a daily dose of from 0.01 to 2000 mg/kg.

8. A use according to claim 6 wherein the medicament is a dosage form suitable for administration of the derivative or salt in a daily does of from 0.01 to 1000 mg/kg.

9. A method for the production of a derivative or salt according to claim 1 comprising at least one of the steps of:
(A) reacting a compound of formula (II)

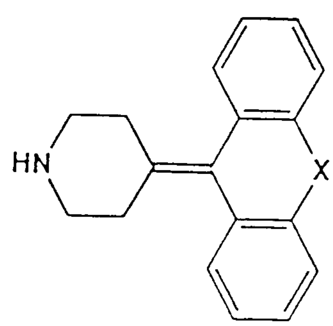

(II)

wherein X is as defined in Claim 1
and a compound of formula (1):

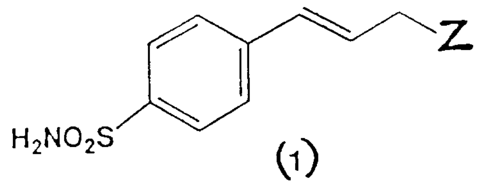

(1)

wherein Z is a leaving group; and
(B) reacting a derivative of formula (I) with an acid, thereby to form a pharmaceutically acceptable salt of the derivative.

**10.** 4-Aminosulfonylcinnamyl alcohol of formula (2)

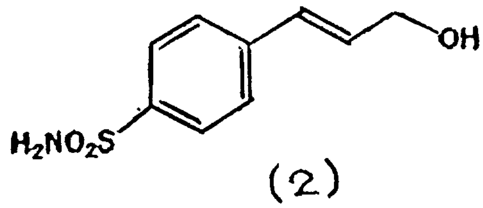

(2)

**11.** A compound of formula (1)

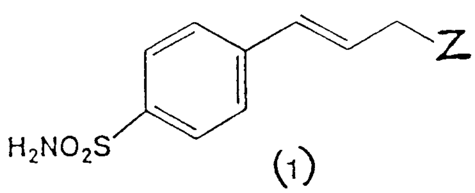

(1)

wherein Z is a leaving group.

## Patentansprüche

**1.** Piperidinderivat der Formel (I):

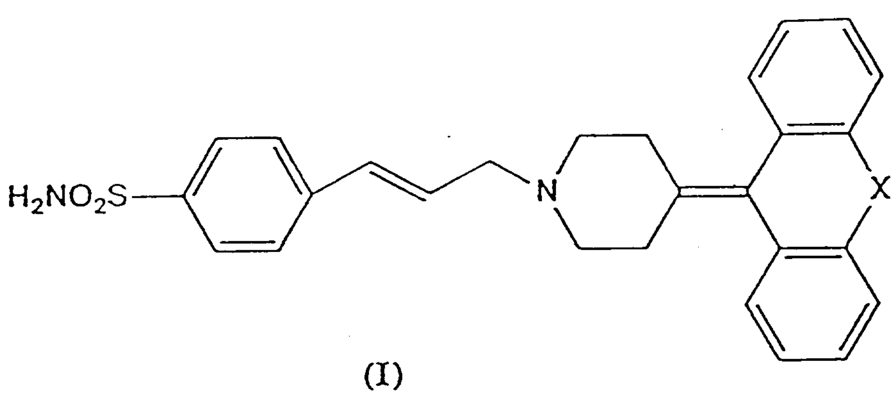

(I)

in der X $-CH=CH-$, $-CH_2CH_2-$ oder $-CH_2S-$ bedeutet,
oder ein pharmazeutisch geeignetes Salz davon.

2. Derivat oder Salz nach Anspruch 1 zur pharmazeutischen Verwendung.

3. Arzneimittelzusammmensetzung, enthaltend ein Derivat oder Salz nach Anspruch 1 und ein pharmazeutisch geeignetes Streckmittel, Verdünnungsmittel oder Trägermaterial.

4. Verwendung eines Derivats oder Salzes nach Anspruch 1 zur Herstellung eines Arzneimittels zur Verhütung oder Behandlung von Bluthochdruck.

5. Verwendung nach Anspruch 4, wobei das Arzneimittel zur oralen Verabreichung bestimmt ist.

6. Verwendung nach Anspruch 4, wobei das Arzneimittel zur Verabreichung auf parenteralem Weg bestimmt ist.

7. Verwendung nach Anspruch 5, wobei das Arzneimittel in einer Dosierungsform vorliegt, die zur Verabreichung des Derivats oder Salzes in einer Tagesdosis von 0,01 bis 2000 mg/kg geeignet ist.

8. Verwendung nach Anspruch 6, wobei das Arzneimittel in einer Dosierungsform vorliegt, die zur Verabreichung des Derivats oder Salzes in einer Tagesdosis von 0,01 bis 1000 mg/kg geeignet ist.

9. Verfahren zur Herstellung eines Derivats oder Salzes nach Anspruch 1, das mindestens einen der folgenden Schritte umfaßt:
(A) Umsetzen einer Verbindung der Formel (II):

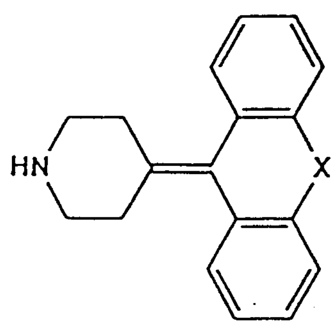

(II)

wobei X wie in Anspruch 1 definiert ist
und einer Verbindung der Formel (1):

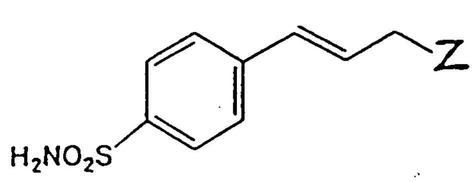

(1)

worin Z eine austretende Gruppe ist, und
(B) Umsetzen eines Derivats der Formel (I) mit einer Säure zur Bildung eines pharmazeutisch geeigneten Salzes des Derivats.

**10.** 4-Aminosulfonylcinnamylalkohol der Formel (2)

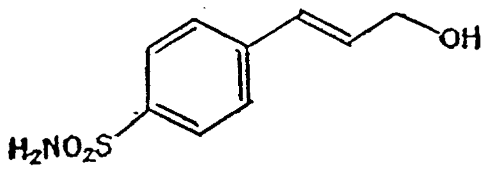

(2)

**11.** Verbindung der Formel (1):

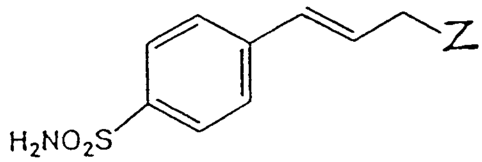

(1)

in der Z eine austretende Gruppe ist.

## Revendications

**1.** Dérivé de pipéridine de formule (I) :

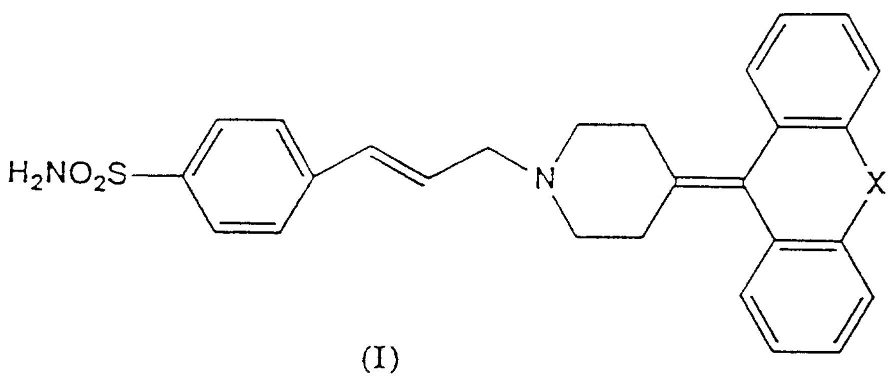

(I)

dans laquelle X est $-CH=CH-$, $-CH_2CH_2-$ ou $-CH_2S-$ ;
ou sel pharmaceutiquement acceptable de ce dernier.

**2.** Dérivé ou sel selon la revendication 1 pour usage pharmaceutique.

**3.** Composition pharmaceutique comprenant un dérivé ou un sel selon la revendication 1 et un excipient, diluant ou véhicule pharmaceutiquement acceptable.

**4.** Utilisation d'un dérivé ou d'un sel selon la revendication 1 dans la préparation d'un médicament destiné à la prévention ou au traitement de l'hypertension.

**5.** Utilisation selon la revendication 4, dans laquelle le médicament est destiné à l'administration orale.

**6.** Utilisation selon la revendication 4, dans laquelle le médicament est destiné à l'administration par une voie parentérale.

**7.** Utilisation selon la revendication 5, dans laquelle le médicament est une présentation convenant à l'administration du dérivé ou du sel en une dose journalière comprise entre 0,01 et 2000 mg/kg.

**8.** Utilisation selon la revendication 6, dans laquelle le médicament est une présentation convenant à l'administration du dérivé ou du sel en une dose journalière comprise entre 0,01 et 1000 mg/kg.

**9.** Procédé de production d'un dérivé ou d'un sel selon la revendication 1, comprenant au moins une des étapes consistant à :

(A) mettre à réagir un composé de formule (II)

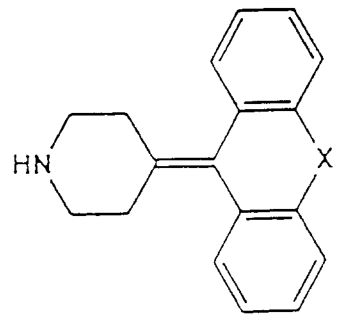

(II)

dans laquelle X est tel que défini à la revendication 1
et un composé de formule (1) :

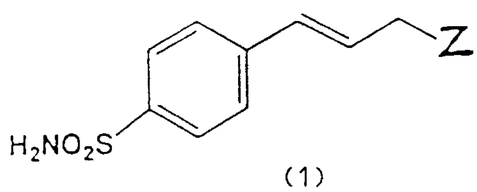

(1)

dans laquelle Z est un groupe qui part ; et

(B) mettre à réagir un dérivé de formule (I) avec un acide pour former un sel pharmaceutiquement acceptable du dérivé.

**10.** Alcool 4-aminosulfonylcinnamylique de formule (2)

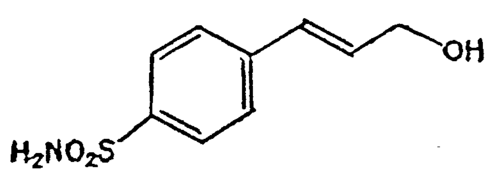

(2)

**11.** Composé de formule (1)

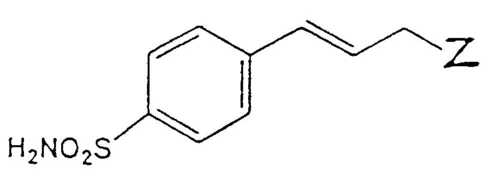

(1)

dans laquelle Z est un groupe qui part.